Europäisches Patentamt

European Patent Office

Office européen des brevets

(11) Veröffentlichungsnummer : **0 129 842**
**B1**

(12) **EUROPÄISCHE PATENTSCHRIFT**

(45) Veröffentlichungstag der Patentschrift :
09.09.87

(51) Int. Cl.⁴ : **C 07 C 43/04, C 07 C 41/06**

(21) Anmeldenummer : 84107085.7

(22) Anmeldetag : 20.06.84

(54) Verfahren zur Herstellung von Methyl-tert.butyläther und Methyl-tert.amyläther.

(30) Priorität : 24.06.83 DE 3322753

(43) Veröffentlichungstag der Anmeldung :
02.01.85 Patentblatt 85/01

(45) Bekanntmachung des Hinweises auf die Patenterteilung : 09.09.87 Patentblatt 87/37

(84) Benannte Vertragsstaaten :
AT BE CH DE FR GB IT LI NL SE

(56) Entgegenhaltungen :
FR-A- 2 381 010
US-A- 4 320 233

(73) Patentinhaber : DEUTSCHE TEXACO AKTIENGESELL-
SCHAFT
Überseering 40
D-2000 Hamburg 60 (DE)

(72) Erfinder : Neier, Wilhelm, Dr.
An der Landwehr 40
D-4134 Rheinberg 3 (DE)
Erfinder : Webers, Werner
Rektor-Horn-Strasse 42
D-4134 Rheinberg 3 (DE)
Erfinder : Dettmer, Michael, Dr.
Königsbergerstrasse 29
D-4134 Rheinberg 1 (DE)

(74) Vertreter : Schupfner, Gerhard D.
Müller, Schupfner & Gauger Karlstrasse 5 Postfach
14 27
D-2110 Buchholz/Nordheide (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

## Beschreibung

Die Erfindung bezieht sich auf ein Verfahren zur Herstellung von Methyl-tert.butyläther und Methyl-tert.amyläther durch Umsetzung von Isobuten bzw. Isoamylen, insbesondere von einem solche Olefine enthaltendem $C_4$- bzw. $C_5$-Kohlenwasserstoffschnitt, mit Methanol in Gegenwart saurer Feststoffkatalysatoren in mehreren Reaktionsstufen.

Bekanntlich wird bei der Synthese von Methyl-tert.butyläther (MTBE) abhängig von den Reaktorbedingungen Dimetyläther (DME) als Nebenprodukt gebildet. Dieser Dimetyläther verbleibt, falls das Reaktionsgemisch mit Wasser gewaschen wird, nach der Wasserwachung und destillativen Aufarbeitung im Produkt-Raffinat. Für die Weiterverarbeitung werden an dieses Raffinat unterschiedliche Anforderungen gestellt. Häufig können höhere DME-Gehalte toleriert werden. Bei anderen Verfahren, z. B. der HF-Alkylierung, kann ein zu hoher DME-Gehalt zu erheblichen Störungen führen. Im Falle der Weiterverarbeitung des Raffinats in einer HF-Alkylierung liegt daher die Anforderung an die Produktreinheit bei max. 10 bis 15 ppm DME im Raffinat.

Bei den bekannten Verfahren liegt der DME-Gehalt im Raffinat bei 100 bis 300 ppm. Die Entfernung von DME aus dem Raffinat macht ein erneutes Strippen des Raffinats erforderlich, siehe Chemistry and Industry, 21. August 1982, Seiten 570 ff. Da dieses Raffinat, falls von einem $C_4$-Kohlenwasserstoffschnitt aus dem Dampf-Cracker oder einer FCC-Anlage ausgegangen wird, neben den $C_4$-Anteilen häufig auch größere Anteile Propan/Propylen (3 bis 10 %) enthält und DME mit Propan ein Azeotrop mit 8 % DME bildet, ist die DME-Abtrennung aufwendig und teuer.

Bei der Synthese von Methyl-tert.amyläther (TAME) wird ebenfalls Dimethyläther (DME) als Nebenprodukt gebildet.

Es bestand daher die Aufgabe, die Synthese von MTBE und TAME so durchzuführen, daß die DME-Bildung so weit unterdrückt wird, daß eine anschließende DME-Entfernung aus dem Raffinat entfallen kann.

Im allgemeinen werden für die Verätherung von Isoolefin mit Methanol Molverhältnisse von Alkohol zu Isoolefin von 0,5 : 1 bis 6 : 1, vorzugsweise 1 : 1 bis 2 : 1 vorgeschlagen. Als Reaktionstemperatur werden 50 °C bis 120 °C, vorzugsweise 70 °C bis 120 °C, und als geeigneter Reaktionsdruck 5 bis 50 bar im Stand der Technik angegeben ; siehe z. B. DE-OS-29 11 077, Seiten 4, 5 und EP-A1-0015 513, Seite 6. Es ist auch bekannt, die ersten Reaktionsstufen zur Abführung der Reaktionswärme mit Kreisläufen zu versehen, siehe SRI-Report 76-1-1, Seiten 5/6.

In der US-PS-4 262 146 wird zur Temperaturregelung eine Aufteilung des Kohlenwasserstoffstroms vorgeschlagen, und zwar wird dort je nach Temperaturverlauf im ersten Reaktor ein Teil der Isoolefinkomponente abgezweigt und in den zweiten Reaktor gegeben.

In allen bekannten Veröffentlichungen wird bisher der DME-Bildung keine Beachtung geschenkt. Man richtet vielmehr die Aufmerksamkeit auf Verfahren zur DME-Abtrennung aus dem Produktstrom.

Versuche haben nun gezeigt, daß bei der üblichen Überschußfahrweise (Verhältnis Methanol : Isoolefin von > 1 : 1) die Äther-Bildung unabhängig vom Molverhältnis ist. Es ist zwar möglich, die DME-Bildung z. B. durch möglichst niedrige Reaktionstemperaturen von > 100 ppm auf etwa 50 ppm abzusenken, wobei allerdings bekanntermaßen der Umsatz stark zurückgeht. Der Restisoolefingehalt liegt dann bei 2,5 %.

In Lösung der gestellten Aufgabe wird ein Verfahren zur Herstellung von Methyl-tert-butyläther und Methyl-tert-amyläther durch Umsetzung von Isobuten bzw. Isoamylen, insbesondere von einem solche Olefine enthaltendem $C_4$- bzw. $C_5$-Kohlenwasserstoffschnitt, mit Methanol in Gegenwart stark saurer Kationenaustauscher in mehreren Reaktionsstufen, bereitgestellt, dadurch gekennzeichnet, daß man bei einem Gesamtmolverhältnis von Methanol zu Isobuten bzw. Isoamylen von 1 : 1 bis 1,2 : 1 in der ersten Reaktionsstufe die gesamte Menge der Olefin-Komponente mit einem Teil des Methanols in einem Molverhältnis Methanol zu Isobuten bzw. Isoamylen von 0,65 : 1 bis zu 0,85 : 1 umsetzt, so daß die Methanolkonzentration des Effluentstromes der ersten Reaktionsstufe höchstens 0,4 Gew.- % beträgt, und sodann in einer weiteren Reaktionsstufe die Restmenge des Methanols zudosiert oder in mehreren weiteren Reaktionsstufen in Teilmengen zudosiert.

Es wurde nämlich überraschenderweise gefunden, daß bei voller Dosierung des Kohlenwasserstoff-Schnittes in den ersten Reaktorabschnitt und bestimmter Aufteilung der Methanoldosierung auf den ersten und zweiten Reaktorabschnitt, besser noch auf den ersten, zweiten und dritten Reaktorabschnitt, die DME-Bildung so reduziert werden kann, daß im Effluent des Reaktors der DME-Gehalt bei bis zu < 5 ppm liegt. Die Methanoldosierung wird so vorgenommen, daß die Methanolkonzentration im Effluent bzw. Recycle-Produkt des ersten Reaktors bei max. 0,4 %, vorzugsweise bei < 0,1 % liegt. Bei dieser Fahrweise, wobei der Recyclefaktor, d. h. Recyclemengen zu Frischdosierung Kohlenwasserstoffschnitt plus Methanol zweckmäßigerweise ≥ 2 beträgt, werden 0,65 bis 0,85, vorzugsweise 0,7 bis 0,85 Mol Methanol pro Mol Isoolefin sogleich eingespeist. Im zweiten Reaktorabschnitt wird wiederum soviel Methanol zudosiert, daß im Effluent dieses Abschnitts die Methanolkonzentration 0,4 %, vorzugsweise 0,1 % nicht übersteigt. Bei dieser Fahrweise können weitere 0,1 bis 0,3 Mol Methanol pro Einsatzisoolefin zudosiert werden. Die restliche Methanolmenge bis zu einem Molverhältnis Methanol zu Isoolefin von 1 : 1 bis 1,2 : 1, vorzugsweise 1,05 bis 1,15 : 1, wird dem Zulaufstrom des letzten Reaktorabschnitts zugegeben.

Bevorzugt erfolgt die Methanoldosierung in den Kreislauf der betreffenden Reaktionsstufe.

Das neue Verfahren läßt sich vorteilhaft bei einem Recycleverhältnis von 2 : 1 bis 10 : 1, vorzugsweise 3 : 1 bis 5 : 1, in den ersten beiden Reaktorabschnitten durchführen.

Die Umsetzung wird bevorzugt bei einer Temperatur von 35 bis 70 °C und einem Druck von 5 bis 50 bar durchgeführt.

Nachdem bei einer Methanolkonzentration im Reaktoreffluent von 1,5 % bis 18 % kein Einfluß auf die DME-Bildung zu erkennen war, ist bei dem angewendeten Verfahren überraschend, daß durch eine weitere Absenkung des Methanolgehaltes auf 0,4 und besser auf 0,1 %, ein deutlicher Rückgang der DME-Bildung beobachtet wurde. Eine Verminderung der Methanoldosierung auf Molverhältnisse von Methanol zu Isoolefin von unter 1 : 1 war aufgrund der Literatur nicht ratsam, siehe u. a. EP-A1-0 071 238, Seite 10, da dann Oligomerisierung bzw. Polymerisation eintritt, die auch zu einer Schädigung des Katalysators führt.

Nach dem erfindungsgemäßen Verfahren wurde auch gefunden, daß bei einem Verhältnis Methanol zu Isoolefin von 0,6 : 1 im ersten Reaktionsabschnitt Oligomere in einer Größenordnung von 3 bis 5 %, bezogen auf MTBE bzw. TAME, gebildet werden. Diese Nebenreaktion steigt mit fallendem Methanolverhältnis weiter an. Überraschenderweise kann diese Oligomerisation bis auf das gleiche Maß reduziert werden (< 0,2 %) wie bei einem Methanolverhältnis > 1 : 1, wenn die Methanoldosierung in den Kreislaufstrom der ersten Reaktorabschnitte so erfolgt, daß ein Methanol zu Isoolefin-Verhältnis zwischen 0,65 bis 0,85 : 1, vorzugsweise 0,70 bis 0,85 erreicht wird, obwohl gleichzeitig die Methanolkonzentration im Effluentstrom bei < 0,1 % liegt.

Das erfindungsgemäße Verfahren wird in den nachfolgenden Beispielen erläutert.

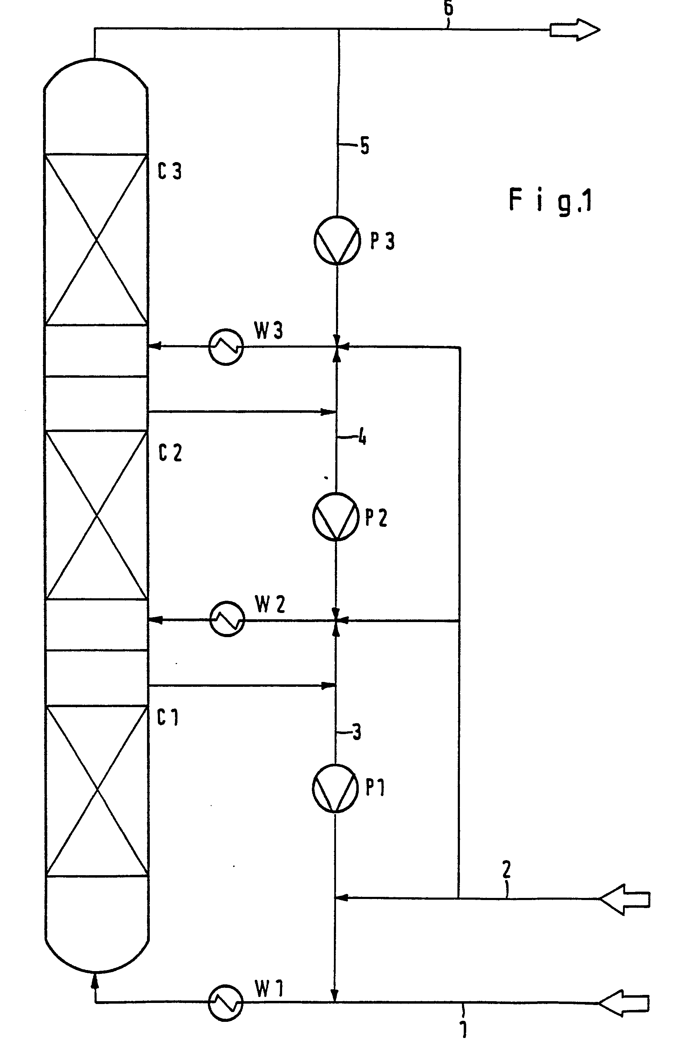

Das in Fig. 1 gezeigte Verfahrensschema besteht aus den drei Reaktorstufen $C_1$ bis $C_3$, die jeweils mit einem Produktionskreislauf 3-5 versehen sind. Die Umlaufmengen werden mit Hilfe der Pumpen $P_1$ bis $P_3$ eingestellt. In jedem Kreislauf befindet sich ein Wärmeaustauscher $W_1$ bis $W_3$ zur Abführung der Reaktionswärme. Das Verfahren kann auch in mehreren hintereinandergeschalteten Reaktoren durchgeführt werden.

Die Versuchsanordnung bestand aus drei Reaktorstufen mit einer lichten Weite von jeweils 26 mm und Längen von 3,0 m, 1,5 m und 3,0 m für die einzelnen Reaktionsstufen. Das Katalysatorvolumen lag bei 1 100 ml, 550 ml und 925 ml. Als Katalysator wurde ein trockener, handelsüblicher stark saurer makroporöser Kationenaustauscher (Amberlyst 15) eingesetzt. (Andere Katalysatoren, z. B. Lewatit SPC 118, Duolite C 26, zeigen in Laborversuchen gleiches Verhalten.)

Vergleichsbeispiel 1

Der Versuch wurde in der beschriebenen Apparatur durchgeführt. Dabei wurden in die Reaktorstufe $C_1$ über Leitung 1 stündlich 4 600 g eines TCC-Gases mit einem Isobutengehalt von 13,2 % und über Leitung 2 390 g Methanol (Molverhältnis Methanol zu Isobuten 1,12 : 1) eingespeist, LHSV = 3,3 $h^{-1}$. In allen drei Reaktorstufen wurde eine maximale Temperatur von 65 °C eingehalten. Mit Hilfe der Pumpen $P_1$ und $P_2$ wurden in den ersten beiden Reaktorstufen eine Recyclemenge von je 20 l/h eingestellt. Die dritte Reaktorstufe wurde ohne Kreislaufführung des Reaktionsproduktes betrieben. Der Reaktionsdruck betrug 10 bar.

Unter diesen Bedingungen wurde Isobuten bis zu einem Restgehalt von 0,7 % im Raffinat (95 %iger Umsatz) umgesetzt. Der Methanolgehalt betrug im Kreislaufprodukt der ersten Reaktorstufe 1,9 %, im Kreislaufprodukt der zweiten Stufe 1,4 % und nach der dritten Stufe 1,2 %. Im Reaktoreffluent wurde ein Gehalt an DME von 190 bis 200 ppm nachgewiesen.

Vergleichsbeispiel 2

Das Vergleichsbeispiel 1 wurde mit der Maßgabe wiederholt, daß die Methanoleinspeisung auf 1 440 g/h erhöht wurde (Molverhältnis Methanol zu Isobuten 4,13 : 1). Alle anderen Bedingungen wurden konstant gehalten. Die LHSV erhöhte sich aufgrund der erhöhten Methanoleinspeisung von 3,3 auf 3,7 $h^{-1}$. Die Methanolkonzentration lag in den einzelnen Reaktorstufen jetzt bei 18,9 % im Kreislaufprodukt der ersten Stufe, bei 18,5 % im Kreislaufprodukt der zweiten Stufe und bei 18,4 % nach der dritten Reaktorstufe. Der Restisobutengehalt lag im Raffinat bei 0,7 %, und der DME-Gehalt betrug 190 bis 230 ppm.

Vergleichsbeispiel 3

Das Vergleichsbeispiel 1 wurde mit folgenden Änderungen wiederholt : Die Temperaturen wurden auf 50 °C in der ersten Stufe und auf 45 °C in den beiden folgenden Reaktorabschnitten abgesenkt. Gleichzeitig wurde die LHSV auf 2,0 $h^{-1}$ und das Molverhältnis Methanol zu Isobuten wieder auf 1,12 : 1 abgeändert, d. h. es wurden 2 850 g/h TCC-Gas und 242 g/h Methanol eingespeist. Die Recyclemengen wurden konstant gehalten. Unter diesen Bedingungen wurde ein Isobutenumsatz von 83 % (2,5 % Isobuten-Restgehalt) und DME-Gehalte im Reaktoreffluent von 45 bis 50 ppm erhalten. Der Anteil an Oligomeren (vorwiegend Di-isobuten) lag bei < 0,2 %, bezogen auf gebildetes MTBE.

Beispiel 1

Das Vergleichsbeispiel 3 wurde mit der maßgabe wiederholt, daß Methanol sowohl in die erste als auch in die zweite Reaktorstufe dosiert wurde. Die Methanolkonzentration im Kreislaufprodukt der ersten Stufe lag dabei bei < 0,1 %, im Kreislaufprodukt der zweiten Stufe bei 1,4 % und im Effluent des Reaktors bei 1,2 %. Dabei wurden stündlich 160 g der Methanolmenge (entsprechend einem Molverhältnis Methanol zu Isobuten von 0,75 : 1) in den Kreislauf der ersten Reaktorstufe und 82 g Methanol in den Kreislauf der zweiten Reaktorstufe dosiert. Es wurde ein Umsatz von 95 % (Restisobutengehalt = 0,8 %) und ein DME-Gehalt von 10 bis 15 ppm im Reaktoreffluent 6 erhalten. Die Bildung von Oligomeren (Di- und Triisobutylen) lag bei < 0,2 %.

Beispiel 2

Das Vergleichsbeispiel 3 wurde mit der Maßgabe wiederholt, daß jetzt die Methanolmenge stündlich von 242 g auf die drei Reaktorstufen wie folgt aufgeteilt wurde : 175 g/h in den Kreislauf der ersten Stufe, 25 g/h Methanol in den Kreislauf der zweiten Stufe und 42 g/h in den Zulauf der dritten Reaktorstufe. Die Methanolgehalte in den Kreislaufprodukten $C_1$ und $C_2$ bzw. dem Reaktoreffluent des Reaktors lag bei < 0,1 % im Kreislauf $C_1$, bei < 0,1 % im Kreislauf der Reaktorstufe $C_2$ und bei 1,1 % nach der dritten Reaktorstufe. Der Isobutenumsatz betrug 96 % (Restisobuten 0,7 %). Die Bildung von Oligomeren lag bei < 0,2 % und der Gehalt an DME im Reaktoreffluent bei 2 bis 5 ppm.

Vergleichsbeispiel 4

Beispiel 1 wurde mit der Maßgabe wiederholt, daß die Dosierung von Methanol in den Kreislauf der ersten Reaktorstufe von 160 g/h auf 125 g/h reduziert wurde, entsprechend einem Molverhältnis Methanol zu Isobuten von 0,58 : 1. Die Methanolkonzentration im Reaktoreffluent betrug < 0,1 %. Weitere 117 g Methanol wurden in den Kreislauf der zweiten Stufe dosiert. Unter sonst gleichen Bedingungen wurde ein Isobutenumsatz von 93 % (Restisobutengehalt = 1,0 %) erhalten. Der DME-Gehalt lag bei 10 bis 15 ppm, der Anteil an Diisobuten plus Oligomeren im MTBE lag jetzt bei 3 bis 5 %.

Vergleichsbeispiel 5

Beispiel 1 wurde in der Weise wiederholt, daß unter sonst gleichen Bedingungen die Methanoldosierung in den Kreislauf der ersten Reaktorstufe auf 193 g/h erhöht wurde, entsprechend einem Molverhältnis Methanol zu Isobuten von 0,9 : 1. Die Methanolkonzentration im Reaktoreffluent betrug 1,6 %. In den Kreislauf der zweiten Stufe wurden weitere 49 g/h Methanol zudosiert. Es wurde ein Isobutenumsatz von 90 % (Restisobutengehalt = 1,5 %) erhalten. Der DME-Gehalt stieg unter diesen Bedingungen auf 30 bis 40 ppm an.

Vergleichsbeispiel 6

In der auf Seite 6 beschriebenen Apparatur wurden Versuche mit einem $C_5$-Schnitt durchgeführt, der 8,5 % Isoamylen (2-Methyl-buten-1 und 2-Methyl-buten-2) enthielt. In die Reaktorstufe $C_1$ wurden stündlich 4 250 g des $C_5$-Schnitts über Leitung 1 und 205 g Methanol über Leitung 2 (Molverhältnis Methanol zu Isoamylen 1,24 : 1) eingespeist, LHSV etwa 2,5 $h^{-1}$. Mit Hilfe der Pumpen $P_1$ und $P_2$ wurde wie oben eine Recyclemenge von je 20 l/h eingestellt. Der Reaktionsdruck betrug 10 bar, die Temperatur 60 °C.

Unter diesen Bedingungen wurde eine 90 %ige Umsetzung von Isoamylen zu TAME erzielt. Der DME-Gehalt betrug 200 ppm.

Beispiel 3

Das Vergleichsbeispiel 6 wurde mit der Maßgabe wiederholt, daß stündlich 125 g Methanol, entsprechend einem Molverhältnis von Methanol zu Isoamylen von 0,76 : 1 der Reaktorstufe $C_1$ zudosiert wurden. Weiterhin wurden stündlich 20 g bzw. 60 g Methanol den Reaktorstufen $C_2$ und $C_3$ zudosiert. Die Temperatur in der dritten Reaktorstufe war auf 50 °C herabgesetzt.

Unter dieser Bedingungen wurde eine 92 %ige Umsetzung von Isoamylen zu TAME erhalten. Der DME-Gehalt betrug 15 ppm.

## Patentansprüche

1. Verfahren zur Herstellung von Methyl-tertbutyläther und Methyl-tert-amyläther durch Umsetzung von Isobuten bzw. Isoamylen, insbesondere von einem solche Olefine enthaltendem $C_4$- bzw. $C_5$-Kohlenwasserstoffschnitt, mit Methanol in Gegenwart stark saurer Kationenaustauscher in mehreren Reaktionsstufen, dadurch gekennzeichnet, daß man bei einem Gesamtmolverhältnis von Methanol zu Isobuten bzw. Isoamylen von 1 : 1 bis 1,2 : 1 in der ersten Reaktionsstufe die gesamte Menge der olefin-Komponente mit einem Teil des Methanols in einem Molverhältnis Methanol zu Isobuten bzw. Isoamylen von 0,65 : 1 bis zu 0,85 : 1 umsetzt, so daß die Methanolkonzentration des Effluentstromes der ersten Reaktionsstufe höchstens 0,4 Gew. % beträgt, und sodann in einer weiteren Reaktionsstufe die Restmenge des Methanols zudosiert oder in mehreren weiteren Reaktionsstufen in Teilmengen zudosiert.

2. Verfahren nach Anspruch 1, dadurch gekennzeichnet, daß man in der ersten Reaktionsstufe mit soviel Methanol umsetzt, daß die Methanolkonzentration des Effluentstroms der ersten Reaktionsstufe höchstens 0,1 Gew. % beträgt.

3. Verfahren nach Anspruch 1 oder Anspruch 2, dadurch gekennzeichnet, daß man in der ersten Reaktionsstufe die Reaktionskomponenten im Molverhältnis Methanol zu Isobuten bzw. Isoamylen von 0,70 : 1 bis 0,85 : 1, umsetzt.

4. Verfahren nach einem der vorhergehenden Ansprüche, dadurch gekennzeichnet, daß das Verhältnis der Recyclemengen zur Frischdosierung $C_4$-Schnitt bzw. C5-Schnitt plus Methanol ≥ 2 beträgt.

5. Verfahren nach einem der vorhergehenden Ansprüche, dadurch gekennzeichnet, daß man in der zweiten Reaktionsstufe den zweiten Anteil Methanol so zudosiert, daß die Methanolkonzentration des Effluentstroms der zweiten Reaktionsstufe ebenfalls höchstens 0,4 Gew. %, vorzugsweise 0,1 Gew. % beträgt.

6. Verfahren nach einem der vorhergehenden Ansprüche, dadurch gekennzeichnet, daß die Methanoldosierung in den Kreislauf der betreffenden Reaktionsstufe erfolgt.

7. Verfahren nach einem der vorhergehenden Ansprüche, dadurch gekennzeichnet, daß man die Umsetzung bei einer Temperatur von 35 bis 70 °C und einem Druck von 5 bis 50 bar durchführt.

## Claims

1. Process for the preparation of methyl tert. butyl ether and methyl tert. amyl ether by reacting isobutene or isoamylene, particularly a $C_4$ or $C_5$ hydrocarbons cut containing such olefins, with methanol in the presence of strongly acidic cation exchange resins in several reaction stages, characterized by using a methanol/isobutene or isoamylene mole ratio totalling 1 : 1-1.2 : 1 and reacting in the first reaction stage the total amount of olefin component with part of the methanol in a methanol/isobutene or isoamylene mole ratio of 0.65 : 1 to 0.85 : 1 such that the methanol concentration in the effluent from the first reaction stage is max. 0.4 %wt., and then feeding the residual amount of methanol in an additional reaction stage or portions of methanol in several additional reaction stages.

2. Process according to claim 1, characterized by using as much methanol in the first reaction stage that the methanol concentration in the effluent from the first reaction stage is max. 0.1 %wt.

3. Process according to claim 1 or 2, characterized by reacting the reaction components in the first reaction stage using a methanol/isobutene or isoamylene mole ratio of 0.70 : 1-0.85 : 1.

4. Process according to any one of the preceding claims, characterized by using a recycle quantity/fresh $C_4$ or $C_5$ cut plus methanol ratio of higher than or equal to 2.

5. Process according to any one of the preceding claims, characterized by feeding the second methanol portion in the second reaction stage such that also the methanol concentration in the effluent from the second reaction stage is max. 0,4 %wt., preferably 0.1 %wt.

6. Process according to any one of the preceding claims, characterized by feeding the methanol to the recycle stream of the respective reaction stage.

7. Process according to any one of the preceding claims, characterized by performing the reaction at a temperature of 35-70 °C and a pressure of 5-50 bar.

## Revendications

1. Procédé pour la préparation de l'éther méthyl tertio-butylique et de l'éther tertio-amyl méthylique par réaction de l'isobutène ou de l'isoamylène, particulièrement d'une coupe d'hydrocarbures $C_4$ ou $C_5$ renfermant de telles oléfines, avec du méthanol à plusieurs étapes en présence d'échangeurs de cations fortement acides caractérisé en ce qu'un rapport molaire total méthanol/isobutène ou isoamylène de 1 : 1 à 1,2 : 1 on fait réagir dans la première zone la quantité complète de l'oléfine avec une partie du méthanol en utilisant un rapport molaire méthanol/isobutène ou isoamylène de 0,65 : 1 à 0,85 : 1, de sorte que l'effluent de la première zone renferme 0,4 % en poids de méthanol au maximum, et qu'on introduit ensuite le reste du méthanol dans une zone ultérieure ou qu'on introduit des parties de méthanol dans plusieurs zones ultérieures.

2. Procédé selon la revendication 1, caractérisé en ce qu'on utilise tant de méthanol dans la première zone réactionnelle que l'effluent quittant la première zone du réacteur renferme 0,1 % en poids de méthanol au maximum.

3. Procédé selon la revendication 1 ou 2, caractérisé en ce qu'on fait réagir dans la première zone du réacteur les composants réactionnels en utilisant un rapport molaire méthanol/isobutène ou isoamylène de 0,70 : 1 à 0,85 : 1.

4. Procédé selon l'une quelconque des revendications précédentes, caractérisé en ce que le rapport des quantités recyclées/charge fraîche de coupe $C_4$ ou $C_5$ et de méthanol est supérieur ou égal à 2.

5. Procédé selon l'une quelconque des revendications précédentes, caractérisé en ce qu'on introduit dans la deuxième zone du réacteur la deuxième portion de méthanol de sorte que l'effluent quittant la deuxième zone renferme également 0,4 % en poids de méthanol au maximum, de préférence 0,1 % en poids.

6. Procédé selon l'une quelconque des revendications précédentes, caractérisé en ce qu'on introduit le méthanol dans le circuit de la zone respective.

7. Procédé selon l'une quelconque des revendications précédentes, caractérisé en ce qu'on effectue la réaction à une température de 35-70 °C et à une pression de 5 à 50 bar.

Fig.1